# EUROPEAN PATENT APPLICATION

(11) **EP 3 095 479 A1**
(43) Date of publication of application: **23.11.2016**
(21) Application number: 15742954.9
(22) Date of filing: 28.01.2015
(51) Int. Cl.: A61M 16/16, A61M 16/00, A61M 16/06

(54) **RESPIRATORY ASSISTANCE DEVICE**

(30) Priority: 31.01.2014 JP 2014016732
(71) Applicant: Metran Co., Ltd., Kawaguchi-shi, Saitama 332-0015 (JP)
(72) Inventor: NITTA, Kazufuku, Kawaguchi-shi Saitama 332-0015 (JP)
(74) Representative: TBK
(86) International application number: PCT/JP2015/052378
(87) International publication number: WO 2015/115489

(57) **Abstract**

A respiratory assistance device 1 is used by a patient having a breathing problem to send gas to be inspired air into the respiratory tract of the patient. Specifically, the respiratory assistance device 1 includes: a blower 11 for taking in gas and sending out the gas into the respiratory tract of the patient; an upstream-side humidifier 10 for humidifying the gas to be introduced into the blower 11 on the upstream side of the blower 11; and a downstream-side humidifier 12 for humidifying the gas sent out from the blower 11 on the downstream side of the blower 11.

This allows for the sufficient humidification of inspired air even when an inspiratory circuit from the blower is short.

## Description

### Technical Field

The present invention relates to a respiratory assistance device.

### Background Art

Sleep apnea syndrome (SAS) is caused by a root of a tongue and a soft palate moving down due to flaccid muscles during sleep and clogging a trachea. A respiratory assistance device including a blower for applying a positive pressure to the respiratory tract is used for this type of patient (see Japanese Patent No. 5358773 and Metran Co., Ltd., [online], Products > Jusmine, [searched on January 27, 2014], Internet (URL: http://www.metran.co.jp/products/products2/190.html)). The respiratory assistance device sends compressed air supplied from the blower into the respiratory tract of the patient as inspired air after the humidification of the air in an inspiratory circuit.

### Summary of Invention

### Technical Problem

However, there is an increasing tendency to downsize such respiratory assistance devices and the inspiratory circuits thereof are being shortened accordingly, thus failing to achieve the sufficient humidification of inspired air.

The present invention has been made in view of the aforementioned problem. It is an object of the present invention to provide a respiratory assistance device capable of sufficiently humidifying inspired air even when an inspiratory circuit from a blower is short.

### Solution to Problem

(1) The present invention is a respiratory assistance device characterized by including: a blower configured to take in gas and send out the gas into a respiratory tract of a user; an upstream-side humidifier configured to humidify the gas to be introduced into the blower on an upstream side of the blower; and a downstream-side humidifier configured to humidify the gas sent out from the blower on a downstream side of the blower.
   According to the present invention, the humidification amount of the gas to be supplied to the respiratory tract of the user can be increased by humidification in the upstream-side humidifier performed in advance of humidification in the downstream-side humidifier. This allows for the sufficient humidification of inspired air even when the inspiratory circuit from the blower is short and humidification by the downstream-side humidifier is thus insufficient.
   Assuming that humidification is performed only by the upstream-side humidifier without providing the downstream-side humidifier to achieve a humidification amount capable of preventing the respiratory tract of the user from drying, dew condensation will occur in the blower. According to the present invention, on the other hand, a humidification amount capable of preventing dew condensation in the blower is first obtained in the upstream-side humidifier, and then a humidification amount capable of preventing the respiratory tract of the user from drying (a humidification amount to cause dew condensation) is obtained in the downstream-side humidifier. Thus, dew condensation is prevented from occurring in the blower.
(2) The present invention is a respiratory assistance device according to (1) described above and characterized in that the blower includes a heater configured to heat up the gas introduced thereinto.
   According to this invention, dew condensation can be prevented from occurring in the blower. This can increase a humidification amount by the upstream-side humidifier. Thus, a humidification amount capable of preventing the respiratory tract of the user from drying can be obtained even when the inspiratory circuit from the blower is short and a humidification amount by the downstream-side humidifier is thus low.
(3) The present invention is a respiratory assistance device according to (2) described above and characterized in that the blower includes an impeller and a motor configured to rotate the impeller, and the motor also serves as the heater.
   According to this invention, there is no need to separately provide a heater, and thus the respiratory assistance device can be further downsized.
(4) The present invention is a respiratory assistance device according to any of (1) to (3) described above and characterized in that the blower is disposed in front of a face of the user.

### Advantageous Effects of Invention

The respiratory assistance devices in the above-described (1) to (4) of the present invention can provide advantageous effects such that the humidification of inspired air can be sufficiently performed even when the inspiratory circuit from the blower is short.

### Brief Description of Drawings

FIG. 1 is a schematic view illustrating a respiratory assistance device according to an embodiment of the present invention.
FIG. 2 is a block diagram illustrating a hardware configuration of a control unit.
FIG. 3 is a block diagram illustrating a functional configuration of the control unit.

### Description of Embodiments

A respiratory assistance device 1 according to an embodiment of the present invention will be described below in detail with reference to the drawings.

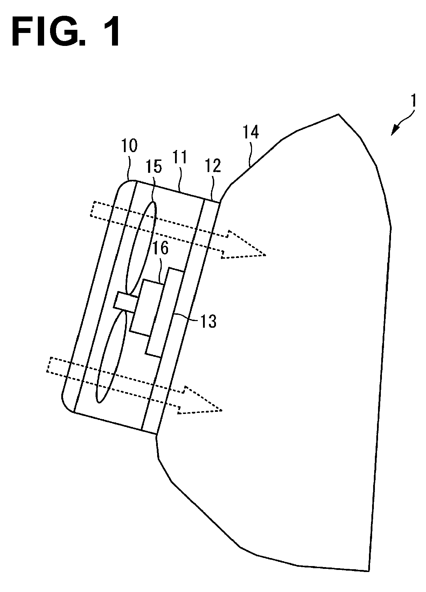

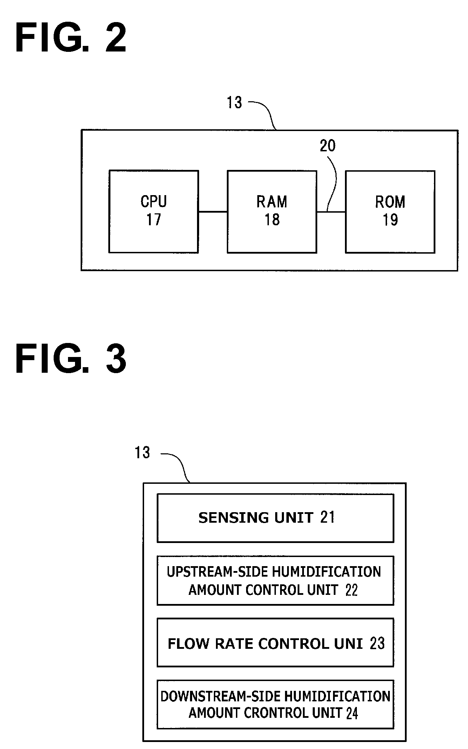

The configuration of the respiratory assistance device 1 according to the embodiment of the present invention will be described first with reference to FIGS. 1 to 3. FIG. 1 is a schematic view illustrating the respiratory assistance device 1. FIG. 2 is a block diagram illustrating a hardware configuration of a control unit 13. FIG. 3 is a block diagram illustrating a functional configuration of the control unit 13. Note that part of the configuration in each of the present and following diagrams is appropriately omitted to simplify the drawings. For example, the illustration of an expiratory circuit such as an expiratory valve is omitted and the description thereof is also omitted.

The respiratory assistance device 1 shown in FIG. 1 is a mask-type device used by a patient (user) having a breathing problem. The respiratory assistance device 1 sends gas (compressed air) to be inspired air into the respiratory tract of the patient. Specifically, the respiratory assistance device 1 includes an upstream-side humidifier 10, a blower 11, a downstream-side humidifier 12, the control unit 13, and a mask 14, for example.

The upstream-side humidifier 10 humidifies gas to be introduced into the blower 11 on the upstream side of the blower 11 so as not to cause dew condensation in the blower 11. Specifically, the upstream-side humidifier 10 includes a container (not shown) to contain water for humidification, and a water-permeable member (not shown) disposed upstream of the blower 11 and used to evaporate the water supplied from the container, for example. The upstream-side humidifier 10 may be of a mask integrated type, which is fixed to the mask 14, or may be of a mask separated type, which includes a container to contain water for humidification provided separately from the mask and connected to the upstream side of the blower 11. For the detail of the upstream-side humidifier 10, refer to the humidifier described in Japanese Patent No. 4771711, for example.

As a result of the blower 11 being fixed to the mask 14, the blower 11 is disposed in front of the face (mouth) of a user. The blower 11 takes in and compresses gas (atmosphere) humidified in the upstream-side humidifier 10, and then sends out the compressed gas into the respiratory tract of the user as inspired air. Specifically, the blower 11 includes an impeller 15, and a motor 16 for rotating the impeller 15, for example. The motor 16 functions also as a heater for heating up gas taken in by the blower 11 due to its heat generation when being driven. In other words, the motor 16 also serves as the heater for heating up gas taken in by the blower 11 to about a body temperature or less (for example, 37 degrees or lower). For the detail of the blower 11, refer to the blower described in Japanese Patent No. 5211302, for example.

The downstream-side humidifier 12 humidifies gas sent out from the blower 11 on the downstream side of the blower 11 so as not to dry the respiratory tract of the user (a degree to cause dew condensation). Specifically, the downstream-side humidifier 12 includes, for example, a container (not shown) to contain water for humidification, and a water-permeable member (not shown) disposed downstream of the blower 11 and used to evaporate the water supplied from the container. The downstream-side humidifier 12 may be of a mask integrated type, which is fixed to the mask 14, or may be of a mask separated type, which includes a container to contain water for humidification provided separately from the mask and connected to the downstream side of the blower 11. For the detail of the downstream-side humidifier 12, refer to the humidifier described in Japanese Patent No. 4771711, for example.

As shown in FIG. 2, the control unit 13 includes a CPU 17, a first storage medium 18, a second storage medium 19, and a bus 20, for example.

The CPU 17 is what is called a central processing unit. The CPU 17 executes various programs to implement various functions of the control unit 13. The first storage medium 18 is what is called a RAM (random access memory) and used as a workspace of the CPU 17. The second storage medium 19 is what is called a ROM (read only memory) and used for storing programs to be executed by the CPU 17. The bus 20 is wiring for connecting the CPU 17, the first storage medium 18, the second storage medium 19, etc., together for communication thereamong.

As shown in FIG. 3, the control unit 13 includes, as its functional configuration, a sensing unit 21, an upstream-side humidification amount control unit 22, a flow rate control unit 23, and a downstream-side humidification amount control unit 24, for example.

The sensing unit 21 constantly obtains, and then transmits to the upstream-side humidification amount control unit 22, sensed data of a hygrometer and a thermometer (these are not shown) disposed upstream of the upstream-side humidifier 10. The sensing unit 21 constantly obtains, and then transmits to the flow rate control unit 23, sensed data of a barometer, a flowmeter, and a thermometer (these are not shown) disposed downstream of the blower 11. The sensing unit 21 also constantly obtains, and then transmits to the downstream-side humidification amount control unit 24, sensed data of a hygrometer and a thermometer (these are not shown) disposed downstream of the blower 11 and upstream of the downstream-side humidifier 12.

The upstream-side humidification amount control unit 22 controls a control signal to the upstream-side humidifier 10 by referring to the sensed data by the sensing unit 21 so that its humidification amount approaches a target humidification amount. For example, the upstream-side humidification amount control unit 22 controls the control signal to the upstream-side humidifier 10 so that 70% of the ultimately required humidification amount is humidified in the upstream-side humidifier 10.

The flow rate control unit 23 controls, by referring to the sensed data by the sensing unit 21, a control signal to the motor 16 of the blower 11 so that its flow rate value approaches a target flow rate value without exceeding a target gas temperature.

The downstream-side humidification amount control unit 24 controls a control signal to the downstream-side humidifier 12 by referring to the sensed data by the sensing unit 21 so that its humidification amount approaches a target humidification amount. For example, the downstream-side humidification amount control unit 24 controls the control signal to the downstream-side humidifier 12 so that 30% of the ultimately required humidification amount, i.e., the amount left after the humidification in the upstream-side humidifier 10, is humidified in the downstream-side humidifier 12.

The flow of inspired air will be described next with reference to FIG. 1.

The operation of the respiratory assistance device 1 causes the rotation of the impeller 15 in the blower 11. This causes gas (atmosphere) to be introduced into the blower 11 via the upstream-side humidifier 10. The gas is humidified in the upstream-side humidifier 10 so as not to cause dew condensation in the blower 11. The gas is compressed in the blower 11. The gas compressed in the blower 11 is sent into the respiratory tract of the user as inspired air via the downstream-side humidifier 10. The gas is humidified in the downstream-side humidifier 10 so as not to dry the respiratory tract of the user.

As described above, the respiratory assistance device 1 can increase the humidification amount of the gas to be supplied to the respiratory tract of the user by humidifying the gas in the upstream-side humidifier 10 in advance of the humidification of the gas in the downstream-side humidifier 12. This allows for the sufficient humidification of inspired air even when the inspiratory circuit from the blower 11 is short and the humidification by the downstream-side humidifier 12 is thus insufficient.

Assuming that humidification is performed only by the upstream-side humidifier 10 without providing the downstream-side humidifier 12 to achieve the humidification amount capable of preventing the respiratory tract of the user from drying, dew condensation will occur in the blower 11. The respiratory assistance device 1, on the other hand, can obtain a humidification amount, in the upstream-side humidifier 10, capable of preventing dew condensation in the blower 11 and then obtain a humidification amount, in the downstream-side humidifier 12, capable of preventing the respiratory tract of the user from drying (a humidification amount to cause dew condensation). Thus, no dew condensation occurs in the blower 11.

As a result of the blower 11 having the heater (motor 16), dew condensation in the blower 11 is prevented from occurring. This can increase the humidification amount by the upstream-side humidifier 10. Thus, the humidification amount capable of preventing the respiratory tract of the user from drying can be obtained even when the inspiratory circuit from the blower 11 is short and the humidification amount by the downstream-side humidifier 12 is thus low.

Since the motor 16 for rotating the impeller 15 also serves as a heater, there is no need to separately provide a heater, thus achieving further downsizing of the respiratory assistance device 1.

The present invention is not limited to the above-described embodiment, but can be variously modified within a range without departing from the general meaning and technical thought thereof.

In other words, the position, size (dimension), shape, material, orientation, and number of each component in the above-described embodiment can be appropriately changed.

Although the above-described embodiment has taken, as an example, the mask-type respiratory assistance device 1, the present invention is not limited thereto. The respiratory assistance device may be of a prong type, for example.

Although the above-described embodiment has taken, as an example, the case where the motor 16 also serves as a heater, the present invention is not limited thereto. A motor and a heater may be provided separately.

### Reference Signs List

- 1: respiratory assistance device
- 10: upstream-side humidifier
- 11: blower
- 12: downstream-side humidifier
- 15: impeller
- 16: motor (heater)

## Claims

1. A respiratory assistance device comprising:
a blower configured to take in gas and send out the gas into a respiratory tract of a user;
an upstream-side humidifier configured to humidify the gas to be introduced into the blower on an upstream side of the blower; and
a downstream-side humidifier configured to humidify the gas sent out from the blower on a downstream side of the blower.

2. The respiratory assistance device according to claim 1, wherein the blower includes a heater configured to heat up the gas introduced thereinto.

3. The respiratory assistance device according to claim 2, wherein
the blower includes an impeller and a motor configured to rotate the impeller, and
the motor also serves as the heater.

4. The respiratory assistance device according to any one of claims 1 to 3, wherein the blower is disposed in front of a face of the user.
